# EUROPEAN PATENT APPLICATION

(11) **EP 4 620 445 A1**
(43) Date of publication of application: **24.09.2025**
(21) Application number: 24184070.1
(22) Date of filing: 24.06.2024
(51) Int. Cl.: A61F 13/534, A61F 13/551

(54) **FOLDED SANITARY NAPKIN**

(30) Priority: 20.03.2024 EP 24164875
(71) Applicant: Ontex BV, 9255 Buggenhout (BE); Ontex Group NV, 9320 Erembodegem (BE)
(72) Inventor: Staelens, Eva, 9900 Eeklo (BE); Descheemaecker, Evan, 9930 Zomergem (BE)
(74) Representative: Saurat, Thibault

(57) **Abstract**

The present disclosure pertains to a sanitary napkin (10) comprising :
- a topsheet (14),
- a backsheet (16),
- an absorbent core (12) arranged between said topsheet (14) and backsheet (16), and
- two wings (24),
wherein the sanitary napkin is arranged in a folded configuration such that the absorbent core is maintained in said folded configuration by the wings (24).

## Description

### TECHNICAL FIELD

The disclosure pertains to the technical field of absorbent articles for hygiene products and the packaging of such absorbent articles. In particular, the present disclosure relates to individually packaged absorbent articles selected from sanitary napkins, adult incontinence pads or panty liners, which are configured to collect and contain blood, menses, urine, vaginal fluids and avoid leakage. The present disclosure also pertains to the packaging process to wrap or enclose such absorbent articles.

### BACKGROUND

Absorbent articles selected from sanitary napkins, adult incontinence pads or pantyliners, used to collect vaginal discharges are well known in the art. Typically, these absorbent articles commonly comprise a topsheet, a backsheet and an absorbent core arranged in-between said topsheet and backsheet and are typically individually enclosed, or packaged, in a wrapper also commonly called a pouch. These absorbent articles are sold in a folded configuration where the absorbent article is folded in two, three or even four with the wrapper securing this folding. These wrappers or pouches are made out of plastic films such as a polypropylene or polyethylene films since these material have several beneficial physical properties such as flexibility, deformability, *etc.* as well as for cost reasons. However, these wrappers are always made from petroleum-derived materials and thus are not environment-friendly.

For example publications such as EP 0 750 896 A2, US6,186,993 B2 or EP 2 532 333 A1 show these conventional pouches where the article is folded multiple times and the packaging wrapper, usually made of polyolefin material, maintains that article folded. There is still a need to improve the environmental impact of these wrappers and offer a solution that involves less plastic without degrading the performance of the packaging of these absorbent articles.

The invention thereto aims to provide an environmentally friendly packaging for absorbent articles selected from sanitary napkins, adult incontinence pads or pantyliners.

### SUMMARY OF THE DISCLOSURE

The present disclosure relates to a sanitary napkin comprising :
- a topsheet,
- a backsheet,
- an absorbent core arranged between said topsheet and backsheet, and
- two wings,
wherein the sanitary napkin is arranged in a folded configuration such that the absorbent core is maintained in said folded configuration by the wings.

The sanitary napkin is arranged in a folded configuration such that absorbent core is maintained directly or indirectly in said folded configuration by the wings. By arranging the absorbent article, or the sanitary napkin, in a way that the wings can maintain said absorbent article, *i.e.* the absorbent core, topsheet and backsheet, in a folded configuration thereby removing the need altogether for wrapper or pouch, the environmental impact of the packaging is lowered since less material is used. In other words, with this arrangement, the absorbent article or sanitary napkin is free of wrapper or is pouch-free.

According to an embodiment, the sanitary napkin extends in longitudinal, transversal and vertical directions, and comprises in a unfolded configuration with respect to the longitudinal direction, a front portion, a rear portion and a central portion arranged between said front and rear portions, the central portion being defined by the length of the wings taken in the point in which they extend from the longitudinal sides of the sanitary napkin in a direction parallel to the longitudinal axis, when in said folded configuration, the front and rear portions are folded onto the central portion, the front and rear portions being arranged between the central portion and the wings with respect to the vertical direction.

For sake of clarity, the sanitary napkin can be either in a partially folded configuration, a fully folded configuration and in an unfolded configuration, in any of these configurations the sanitary napkin extends in longitudinal, transversal and vertical directions (L,T,V). However, it is the wings that maintains the sanitary napkin in a folded state or configuration.

According to an embodiment, each wing comprises an area of adhesive arranged on the side of the backsheet opposite to the absorbent core. Namely the sanitary napkin comprises a layer of adhesive arranged on the side of the backsheet opposite to the absorbent core, said layer of adhesive comprises a first area of adhesive arranged in the main body region and a second area of adhesive arranged on the wings.

According to an embodiment, the sanitary napkin comprises a release liner covering both areas of adhesives arranged on the wings.

According to an embodiment, the wings are at least partially overlapping when folded onto the front or rear portion, preferably said overlapping portion comprising adhesive to attach one wing to the other.

According to an embodiment, a layer of adhesive is arranged on the side of the backsheet opposite to the absorbent core, a release paper being applied onto said layer of adhesive in a main body region arranged inboard of the wings with respect to the transversal direction.

According to an embodiment, the release paper and/or release liner comprises a paper layer comprising cellulosic fibers and a coating comprising a peeling agent selected from silicon resin series, fluororesin series, octadecilisocyanate series wherein said paper layer comprises a basis weight comprised between 10 gsm and 80 gsm, preferably between 12 and 60 gsm, more preferably between 14 and 40 gsm, even more preferably between 15 and 35 gsm.

According to an embodiment, the release paper or the release paper and release liner comprises a paper layer comprising cellulosic fibers and a coating comprising a peeling agent selected from silicon resin series, fluororesin series, octadecilisocyanate series wherein said paper layer comprises a basis weight comprised between 10 gsm and 80 gsm, preferably between 12 and 60 gsm, more preferably between 14 and 40 gsm, even more preferably between 15 and 35 gsm.

According to an embodiment, wherein in a folded configuration the release paper and wings cover between 50 and 100 % of the backsheet located in a main body region of the sanitary napkin arranged inboard of the wings (24) with respect to the transversal direction..

According to an embodiment, in a folded configuration the release paper and wings cover between 50 and 100 % of the surface area of the folded sanitary napkin 10, namely of the main body region of the sanitary napkin. In other words, in a folded configuration, the release paper and wings combined overlay or cover between 50 and 100 % of the backsheet, the backsheet located in the main body region of the sanitary napkin. In other terms, between 0 and 50 % of the backsheet located in the main body region is apparent, meaning visually apparent, i.e. not covered, since the rest of the backsheet located in the main body region is covered by either the wings or the wings or both. Preferably, the total surface areas of the release paper and wings combined are between 60 and 99% % of the surface area of the folded sanitary napkin 10, or between 1 and 40 % of the backsheet located in the main body region is apparent, meaning visually apparent, i.e. not covered by the wings and release paper. More preferably the total surface areas of the release paper and wings combined cover between 70 and 98% % of the surface area of the folded sanitary napkin 10, or between 2 and 30 % of the backsheet located in the main body region is apparent, meaning visually apparent, i.e. not covered.

According to an embodiment, the ratio between the total surface area of the release paper and the total surface area of the first area of adhesive is comprised between 1.05 and 2.5.

According to an embodiment, the ratio between the total surface area of peeling agent arranged on the release paper and/or release liner and the total surface area of adhesive arranged on the backsheet is comprised between 1 and 1.5.

The present disclosure also pertains to a container comprising a plurality of folded sanitary napkin as described herein.

While these sanitary napkins use less material and thus are environmentally friendly, conventional pouches are also serves as a protection against contaminants and moisture for the sanitary napkin. With such folding, this protection is slightly lowered. While not so necessary to have such protection when staying at home, sanitary napkins being sold in cardboard boxes or plastic bags a user can leave them in their package and grab one when needed, when traveling it can be beneficial to have a reusable container such as a small bag, a paperbag or a metal box to carry a limited number of sanitary napkins, e.g. one to five napkins. Hence the present disclosure also pertains to a container comprising a plurality of assemblies comprising a folded sanitary napkin. The container is preferably dimensioned to carry a limited number of sanitary napkins such as one, two, three or four napkins. The present disclosure can also pertains to containers containing as much as 10, 20 or even more sanitary napkins, for example for when traveling on a longer trip, e.g. a three-month trip.

The present disclosure also pertains to a method suitable for maintaining a sanitary napkin in a folded configuration and forming an assembly as described herein, the method comprising:
- folding the front portion onto the central portion and folding the rear portion onto the front portion ; or
- folding the rear portion onto the central portion and folding the front portion onto the rear portion; and
- folding the wings onto the folded front and rear portions.

According to an embodiment, the method comprises a further step of applying a release liner onto both areas of adhesive arranged on the wings.

According to an embodiment, the method further comprises an additional step of printing a graphic element on said backsheet or release paper or release liner.

The present disclosure also pertains to a container comprising a plurality of folded sanitary napkin as described herein, said container being selected from a reusable bag, a paperbag, a cardboard box or a metal box.

All of these embodiments mentioned above can be taken individually or in combination. Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**Fig. 1** illustrates a schematic cross section of an absorbent article;
**Fig. 2** shows a schematic top view of a sanitary napkin according to an embodiment;
**Fig. 3** depicts a schematic perspective view of a sanitary napkin according to an embodiment;
**Fig. 4** represents a schematic perspective view of a sanitary napkin according to another embodiment;
**Fig. 5** shows a schematic perspective view of a sanitary napkin according to another embodiment;
**Fig. 6** illustrates schematic perspective view of a sanitary napkin according to another embodiment;
**Fig. 7** (7A to 7E) depicts a schematic top view of a method of folding a sanitary napkin;
**Fig. 8** shows a schematic top view of a sanitary napkin according to an embodiment;

### DETAILED DESCRIPTION OF THE INVENTION

The present invention concerns the packaging of individually packaged absorbent articles selected from sanitary napkins, adult incontinence pads or pantyliners.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The recitation of numerical ranges by endpoints includes all numbers and fractions subsumed within that range, as well as the recited endpoints.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

"Absorbent article" refers to devices that absorb and contain liquid, and more specifically, refers to devices that are placed against or in proximity to the body of the wearer to absorb and contain the various exudates discharged from the body. Absorbent articles preferably comprise a longitudinal axis defining a length and a transversal axis defining a width, said transversal axis being perpendicular to said longitudinal axis as well as a vertical axis defining a height or thickness. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious topsheet, a backsheet joined to the topsheet, and an absorbent core positioned and held between the topsheet and the backsheet. The topsheet is operatively permeable to the liquids that are intended to be held or stored by the absorbent article, and the backsheet may or may not be substantially impervious or otherwise operatively impermeable to the intended liquids. The absorbent article may also include other components, such as acquisition distribution layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface. More particularly, the present disclosure pertains to absorbent articles in relation to feminine hygiene, or more specifically individually packaged absorbent articles selected from sanitary napkins, adult incontinence pads or panty liners, which are configured to collect and contain blood, menses, urine, vaginal fluids and avoid leakage. Even more particularly, the present disclosure pertains to individually packaged absorbent articles with wings such as sanitary napkins.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common in feminine hygiene articles to combine cellulosic fluff pulp with superabsorbent polymers in an absorbent material or to use cellulosic fluff pulp alone.

"Acquisition distribution layer", "ADL" or "surge management portion" refers to a sub-layer which preferably is a nonwoven wicking layer arranged directly under the topsheet of an absorbent product, which speeds up the transport and improves distribution of fluids throughout the absorbent core. The surge management portion has the ability to quickly collect and temporarily hold liquid surges, and to transport the liquid from its initial entrance point to other parts of the absorbent structure, particularly the retention portion. This configuration can help prevent the exudates from pooling and collecting on the portion of the absorbent garment positioned against the wearer's skin.

The term "adhesive" as used herein is intended to refer to any suitable hot melt, water or solvent borne adhesive that can be applied to a surface of a film layer in the required pattern or network of adhesive areas to form the film-nonwoven laminate of the present disclosure. Accordingly, suitable adhesives include conventional hot melt adhesives, pressure-sensitive adhesives and reactive adhesives (i.e., polyurethane). The absorbent article can comprise different kinds of adhesive. For example, the absorbent article can comprise hot melt adhesive for bonding the topsheet to the backsheet and non-structural or pressure sensitive adhesive for bonding the absorbent article to the garment.

As used herein, the term "non-structural adhesive" refers to any adhesive with a low bonding strength usually demonstrating a load-carrying capacity of less than 1000 pound-force per square inch (psi). Such non-structural adhesives comprise for example vinyls, polychloropene, styrene block copolymer and polyurethane.

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used therein, the term "associated" encompasses configurations in which topsheet is directly joined to backsheet by affixing topsheet directly to backsheet, and configurations wherein topsheet is joined to backsheet by affixing topsheet to intermediate members which in turn are affixed to backsheet. Topsheet and backsheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix topsheet to backsheet. It should be readily appreciated that the above-described attachment means may also be employed to interconnect and assemble together the various other component parts of the article described herein.

The term "backsheet" or "backsheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapor to escape from the absorbent material, while still preventing liquids from passing through. Examples of breathable backsheet materials are porous polymeric films, nonwoven laminates of spunbond and meltblown layers and laminates of porous polymeric films and nonwoven materials.

As used herein, the term "basis weight" refers to an average weight, or a range of weight, of the component in question, namely the layer of cellulosic fibers or the layer of peeling agent, per square meter. Term such as "grammage" is equivalent to "basis weight" and is expressed in grams per square meter, g/m² or gsm.

For instance, the basis weight of the layer of cellulosic fibers is preferably measured according to the ISO 536 standard or any standard method. The amount of peeling agent or sealing agent on the wrapper can be measured by taking a sample of the wrapper where the peeling agent or the sealing agent is arranged, weighing said sample before and after removal of the peeling agent or sealing agent (in grams) the difference giving the weight of the peeling agent or sealing agent, and dividing the weight of peeling agent or sealing agent by the area of the sample (in m2) thereby obtaining the amount of peeling agent or sealing agent in gsm.

As used herein, the term "body-facing", "skin-facing" or "bodyside" side or surface means that surface of the article or component which is intended to be disposed toward or placed adjacent to the body of the wearer during ordinary use, while the "outward", "outward-facing" or "garment-facing" side or surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

As used herein, the term "cellulosic" or "cellulosic fibers" is meant to include any material having cellulose as a major constituent, and specifically comprising at least 50 percent by weight cellulose or a cellulose derivative. Thus, the term includes cotton, typical wood pulps, nonwoody cellulosic fibers, kraft paper, cellulose acetate, cellulose triacetate, rayon, thermomechanical wood pulp, chemical wood pulp, debonded chemical wood pulp, milkweed, or bacterial cellulose.

As used herein "configuration", "conformation", "state" are synonymous and interchangeable and means an arrangement of an element, such as the sanitary napkin, in a particular form, figure, or combination.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "garment" means any type of apparel which may be worn. This includes diapers, training pants, incontinence products, surgical gowns, industrial workwear and coveralls, undergarments, pants, shirts, jackets and the like.

The term "graphic" or "graphic element" includes, but is not limited to, any type of design, image, mark, figure, codes, words, patterns, or the like.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", "bond", "bonded", "affixed", "associated", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, cellulosic material or the like. A layer can be liquid and air permeable, permeable to air but impermeable to liquids, impermeable both to air and liquid, or the like. When used in the singular, it can have the dual meaning of a single element or a plurality of elements. In other terms, a "layer" refers to a single material or a plurality of materials forming a sheet with a given thickness and extending in length and width. The material(s) forming the layer can be arranged in a continuous or discontinuous area, region or zone.

The term "longitudinal", "transversal" and "vertical" as used herein are with respect to the wrapper in an assembled state or in a disassembled or partially assembled state. In other word, the wrapper defines the longitudinal, transversal and vertical directions. The term "in the direction" as used herein means along that direction, e.g. in the longitudinal direction means along the longitudinal direction. With reference to the wrapper, a length corresponds to the dimension extending in the longitudinal direction and is preferably longer than the width, said width being the dimension extending in the transversal direction. The terms "top", "bottom", "upper" and "lower" are all in reference to said vertical direction.

All length, width, and height or thickness can measured with a caliper preferably a micrometer caliper gauge by taking the largest distance separating two points along one direction as defined herein for a given element, e.g. the sanitary napkin in a folded state, the wrapper or the upper strip or the lower strip, or in other words, measuring the distance between two edges of said element along one direction.

The term "length", "width" and "thickness or height" as used herein are respectively in reference to the longitudinal, transversal and vertical direction as defined herein.

"Liquid" means a nongaseous substance and/or material that flows and can assume the interior shape of a container into which it is poured or placed.

The term "nonwoven fabric", "nonwoven layer" or "nonwoven web" means a sheet material having a structure of individual fibers or threads which are interlaid, but not in a regular manner such as occurs with knitting or weaving processes. Nonwoven fabrics, layers or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp" refers to a material made up of cellulose fibers, or cellulosic fibers. The fibers can be either natural or synthetic, or a combination thereof. "Pulp fluff" or "fluff" or "fluff pulp" refers to a material made up of cellulose fibers made from softwood. The material is typically lightweight and has absorbent properties.

By "channels" or "embossments", it is meant that the structure referred to (e.g. the absorbent core) comprises recessed regions forming visible conduits or passages typically extending along the longitudinal axis of the core and having a depth in a direction perpendicular to said longitudinal axis. By "visible" it is herein intended clearly visible by naked eye and typically that the channels have a width generally greater than 1mm, preferably from 5mm to 50 mm, more preferably from 8mm to 40mm, more preferably from 10mm to 30mm, even more preferably from greater than 10mm to less than 25mm.

By "substantially", it is meant at least the majority of the structure referred to.

Use of the term "substrate" includes, but is not limited to, woven or nonwoven webs, porous films, ink permeable films, cellulosic pulp, paper, composite structures, or the like.

Superabsorbent materials suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The topsheet material may further be composed of two fibers, which may be bonded to each other in a bonding pattern. The topsheet fabrics may be composed of a substantially hydrophobic material, and the hydrophobic material may optionally be treated with a surfactant or otherwise processed to impart a desired level of wettability and hydrophilicity.

The term "release liner" and "release paper" are equivalent as used herein, these terms mean a removable paper layer that is coated with peeling agent, such as silicone and arranged to cover or protect a layer of adhesive. For sake of clarity, release liner is in reference to the wings whereas release paper is in reference to the main body region.

The term "bioplastic" used herein refers to any polymeric material, or plastic material, that is biobased, biodegradable/compostable or both. In other words, the term "bioplastic" refers to materials that are either bio-sourced plastics, i.e. derived from biomass, or biodegradable plastics, including compostable plastics. The biodegradable and/or compostable plastics include the plastics derived from fossil resources petrochemical reactions. Some bioplastics have both features bio-sourced and biodegradable.

The term "biodegradable" as used herein refers to a material that when disposed of after use will physically and biologically decompose using known degradation procedures including aerobic, anaerobic, and microbial digestion processes within a specified timeframe, leaving no toxic substances or residue.

The term "compostable" as used herein refers to a material that has the ability to biodegrade under industrial composting conditions including aerobic, anaerobic, and microbial digestion processes within a specified timeframe, leaving no toxic substances or residue.

Embodiments according to the disclosure will now be described. It is understood that technical features described in one or more embodiments maybe combined with one or more other embodiments without departing from the intention of the disclosure and without generalization therefrom.

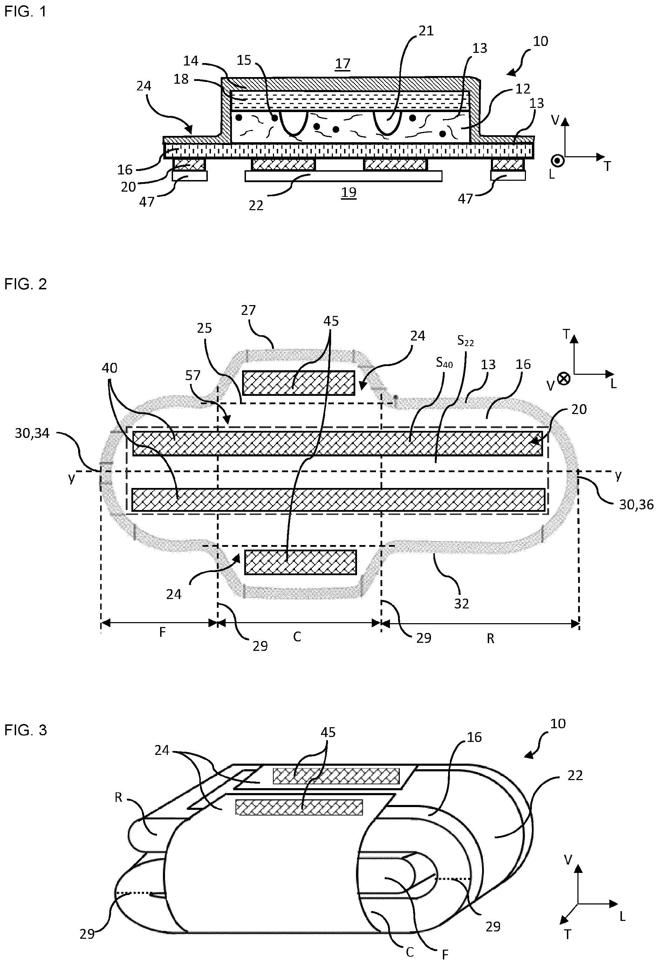

As illustrated in Fig.1, an absorbent article 10, for example a sanitary napkin 10, or a sanitary pad, or a sanitary towel, according to the present disclosure comprises an absorbent core 12 arranged between a liquid permeable topsheet 14 and a liquid impermeable backsheet 16. An optional acquisition distribution layer 18, or surge layer, also called ADL 18, can be positioned between said topsheet 14 and said absorbent core 12. The absorbent core 12 comprises absorbent material. The backsheet 16 is joined to the topsheet 14 with the absorbent core 12, and the optional ADL 18, being positioned and enclosed between the topsheet 14 and the backsheet 16. As illustrated in Fig. 1, the topsheet 14 and the backsheet 16 are associated with one another. The topsheet 14 and backsheet 16 are associated, or joined at the periphery of the sanitary napkin 10 as illustrated in FIG. 2 in bonding regions 13 by attachment means such as an adhesive, mechanical bonds, sonic bonds, thermal bonds, in other words, the bonding regions 13 define the outline or perimeter of the sanitary napkin 10. The topsheet 14 is arranged on the body-facing side 17 whereas the backsheet 16 is arranged on the garment-facing side 19 of the sanitary napkin 10. Optionally, the absorbent core 12 and/or the optional ADL 18, can comprise at least one channel 21 or at least one embossment 21. As illustrated in FIG. 1, the absorbent core 12 comprises two channels 21 or two embossments 21. The topsheet 14 and ADL 18 can be embossed as well, separately or together with the absorbent core. For example, the absorbent article 10 can comprise a topsheet 14 and ADL 18 embossed and a non-embossed absorbent core 12.

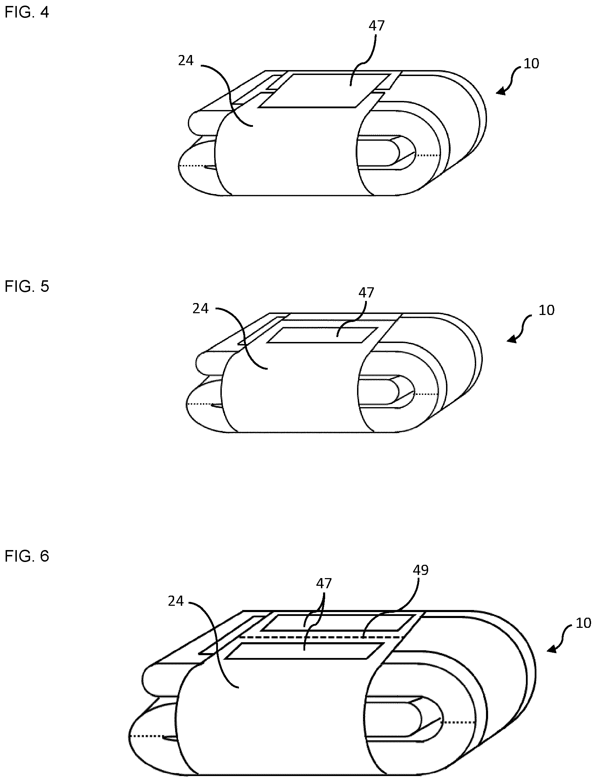

The sanitary napkin 10 comprises on the garment-facing side 19 of the backsheet 16 at least one layer of adhesive 20 so that the sanitary napkin 10 can adhere onto a garment or undergarment, said at least one layer of adhesive 20 ensuring that the absorbent article 10 stays fastened and secured on the underwear. In order to maintain the structural integrity of the adhesive before being used, the sanitary napkin 10 comprises a release paper 22 and release liner(s) 47 corresponding to a peelable layer destined to protect the at least one layer of adhesive 20. The release paper 22 is preferably a siliconized paper acting as a release liner. In other words, the release paper 22 comprises a paper layer comprising cellulosic fibers, meaning paper, and a peeling layer or coating comprising a peeling agent selected from silicon resin series, fluororesin series, octadecilisocyanate series. In other words, the release paper 22 and release liner(s) 47 are a paper layer coated with a silicone derivative. According to an embodiment, the peeling layer comprises a basis weight comprised between 0,3 gsm and 3 gsm, preferably between 0,5 gsm and 1 gsm and the paper layer comprises a basis weight comprised between 10 gsm and 80 gsm, preferably between 12 and 60 gsm, more preferably between 14 and 40 gsm, even more preferably between 15 and 35 gsm. These ranges of basis weights are well adapted as it ensures that the release liner 47 is strong enough to maintain the absorbent article 10 folded. Indeed the wings 24, in an embodiment as illustrated in FIG. 3, if left as such will revert to an unfolded configuration pushed by the front and rear portions, F,R and hence a basis weight greater than 10 gsm ensures that the release liner 47 can resist the force imparted onto the wings by the front and rear portions, F,R and maintain the wings 24 attached as illustrated in FIG. 4. Since the objective is to use less raw material, the basis weight is preferably lower than 80 gsm. This release paper 22 and/or release liner 47 is recyclable. The adhesive can be applied in bloc and/or in stripes and/or in dots and/or in swirls. The at least one layer of adhesive 20 is applied in a pattern that is described hereunder.

A sanitary napkin 10 is illustrated in FIG. 2 in an unfolded configuration, e.g. laid flat on a surface. The sanitary napkin 10 extends in the longitudinal, transversal and vertical directions L, T, V as illustrated in FIG. 1 and 2, thereby defining a length in the longitudinal direction L, a width W in the transversal direction T and a thickness or height in the vertical direction V. In an unfolded configuration as shown in FIG. 2, the length of the sanitary napkin 10 is greater than the width.

The sanitary napkin 10 comprises a topsheet 14, a backsheet 16, an absorbent core 12 arranged between said topsheet 14 and backsheet 16, and two foldable wings 24 or flaps 24 arranged at opposite sides of the sanitary napkin 10 with respect to the transversal direction T. As illustrated in FIG. 2, the sanitary napkin 10 extends in longitudinal, transversal and vertical directions L,T,V, the sanitary napkin has a longitudinal centerline, or central axis, y-y, a pair of transverse sides 30, meaning a pair of sides arranged at each longitudinal ends or longitudinal extremities, of the sanitary napkin 10, and a pair of longitudinal sides 32 extending therebetween, meaning a pair of sides arranged along each transversal ends of the sanitary napkin 10 and connecting the transverse sides 30 to one another. Similarly, the pair of transverse sides 30 connects the two longitudinal sides 32 to one another. In other words, the sanitary napkin 10 comprises two ends, or extremities, at least partially extending in the transversal direction T, *i.e.* the transversal sides 30, and two ends at least partially extending in the longitudinal direction L, *i.e.* the longitudinal sides 32. As illustrated in FIG. 2, the transversal sides 30 are arcuate whereas the longitudinal sides 32 are linear. The sanitary napkin 10 has a front edge 34 and a rear edge 36 positioned on opposite apexes of said transverse sides 30, meaning at each longitudinal extremities of the sanitary napkin 10. The sanitary napkin 10 is divided with respect to the longitudinal direction L, into three sections or portions, a front portion F, a rear portion R and a central portion C arranged between said front and rear portions F,R. The sanitary napkin 10 also comprises wings 24, or flaps 24, arranged at each transversal end of the sanitary napkin 10, the wings 24 extend transversely beyond the longitudinal sides 32 of the sanitary napkin 10. In other words, the flaps 24 are transversally, or laterally, outboard of the longitudinal centerline y-y and central portion C of the sanitary napkin 10. In other terms, each flap 24 comprises a proximal end 25 and a distal end 27 with respect to the longitudinal centerline y-y and with respect to the transversal direction T. Said proximal end 25 is connected to the sanitary napkin 10, *i.e.* connecting the sanitary napkin 10 and the wing 24 and said distal end 27 is the point furthest from the longitudinal centerline y-y. As used herein the term "central portion" C refers to the portion of the sanitary napkin 10 arranged in between the front and rear portions F, R and in between the proximal ends 25 of the flaps 24, meaning intermediate, particularly laterally intermediate of the two wings 24. In other words, the central portion C is defined by the length of the wings 24 taken in the point in which they extend from the longitudinal sides 32 of the sanitary napkin 10 in a direction parallel to the longitudinal axis y-y. In other words, the central portion C is delimited longitudinally by the front and rear portions F, R and transversally by the proximal ends 25 of the flaps 24. The wings 24 may be comprised of an integral and contiguous extension of the topsheet 14, the backsheet 16, or a laminate of both. Alternatively, the wings 24 may be made of separate and independent pieces of material joined to the longitudinal sides 32 of the sanitary napkin 10. Each wing 24 has one face generally coextensive of the topsheet 14 and a mutually opposed face generally coextensive of the backsheet 16.

As illustrated in FIG. 2, the sanitary napkin 10 comprises two regions, a main body 57 and the wings 24. The main body 57 is the region, or portion of the absorbent article 10 comprising the absorbent core 12 and the eventual ADL 18 and is oblong or oblong-shaped, meaning comprising a shape, e.g. a rectangular, that is longer than it is wide and whose corners are rounded. The wings 24 correspond to rectangular or trapezoidal extensions outboard of the main body 57, meaning outboard of the oblong shape, with respect to the transversal direction T. The wings 24 are inboard of the main body 57 with respect to the longitudinal direction L and are arranged in the central portion C of the sanitary napkin 10 with respect to the longitudinal direction L. The main body 57 can be rectangular. The wings 24, only comprising a laminate of topsheet 14 and backsheet 16, are flexible and foldable, meaning the wings 24 can be easily pivoted or rotated at least around a longitudinal axis. The topsheet 14 and the backsheet 16 are associated with one another along the periphery of the sanitary napkin 10 at the main body 57 region and at the wings 24 in bonding regions 13. The pair of longitudinal sides 32 are in reference to the main body 57.

The wings 24 preferably comprises means for attaching one face of the wing 24 to the wearer's undergarment or to the other wing 24. The wings 24 comprises at least one layer of adhesive 20. The attachment means may be pressure sensitive adhesive. If pressure sensitive adhesive is elected, it should be disposed on the face of the wing generally coextensive of the backsheet 16 so that when the wing 24 are wrapped around the crotch portion of the wearer's undergarment, the layer of adhesive will contact the outside of the wearer's undergarment. The sanitary napkin 10 for example comprises a generally rectangular patch of adhesive on each wing 24.

The sanitary napkin 10 comprises at least one layer of adhesive 20 to fasten the sanitary napkin 10 to an undergarment. In order to protect the layer of adhesive 20 prior to its use, the sanitary napkin 10 comprises a release paper 22 and/or release liner(s) 47 as illustrated in FIG. 1, to cover directly or indirectly said at least one layer of adhesive 20 each release paper 22 and/or release liner(s) 47 preferably comprising a layer or coating of peeling agent. Said at least one layer of adhesive 20 is arranged on one side of the backsheet 16, namely on the garment-facing side 19 of the backsheet 16, the backsheet 16 being arranged between the layer of adhesive 20 and absorbent core 12 as illustrated in FIG. 1. In other words, the at least one layer of adhesive 20 is arranged between the backsheet 16 and the release paper 22 and/or release liner(s) 47.

The at least one layer of adhesive 20 is arranged on the backsheet 16 in a discontinuous pattern of adhesive wherein said pattern of adhesive comprises a first area of adhesive 40 arranged in main body 57 region and a second area of adhesive 45 arranged in the wings 24 region. As illustrated in FIG. 2, the layer of adhesive 20 is arranged in a discontinuous pattern of adhesive comprising a first area of adhesive 40 comprising here two stripes of adhesive material 40 both extending longitudinally and parallel in the front, central and rear portions F, C, R in the main body 57 region and a second area of adhesive 45 comprising a strip of adhesive material 45 being arranged on each wing 24. The second area of adhesive 45 is arranged between the proximal and distal ends 25,27 of each wing 24. More specifically, the first area of adhesive 40 is arranged on the main body 57, i.e. on the backsheet at the main body 57 portion and the second area of adhesive 45 is arranged on the wings 24 which are outboard of the main body 57 with respect to the transversal direction T. The first area of adhesive and/or the second area of adhesive, comprising preferably pressure sensitive adhesive can be applied continuously or discontinuously, in blocs or stripes, in dots or in swirls or any kind of common application, such as in triangle, stars, *etc.*

Sanitary napkins 10 are sold in a folded state, either in a three-time fold, meaning comprising two folding lines or that the sanitary napkin has been folded twice, or in a two-time fold, meaning comprising one folding line or that the sanitary napkin has been folded once. It is possible to also have sanitary napkins 10 sold in a four-time fold comprising three folding lines. Such folding enables to fit more articles in a rectangular carboard box or plastic bag.

A sanitary napkin 10 comprising a three-time fold is such that the absorbent article 10 is folded at two folding lines 29 delimiting, or separating, the front, central and rear portions F,C, R. Both front and rear portions F,R are folded inwardly, sequentially or simultaneously, towards the central portion C, each around a folding line 29 to obtain a sanitary napkin 10 in a folded configuration. In order to maintain this article in a folded configuration, the wings 24 are then folded around the proximal end 25 onto the folded front and rear portions F,R. As illustrated in FIG. 3, the sanitary napkin 10 is arranged in a final or fully folded configuration. The folded sanitary napkin 10 extends in longitudinal, transversal and vertical directions (L,T,V), the front and rear portions (F,R) are folded onto the central portion (C), the front and rear portions (F,R) being arranged between the central portion and the wings 24 with respect to the vertical direction V. As illustrated in FIG. 3, the front portion F is folded directly onto the central portion and the rear portion R is folded directly onto the front portion F and thus indirectly onto the central portion C. The present disclosure is not limited to this arrangement and the rear portion R can be folded directly onto the central portion C and the front portion F can be folded directly onto the rear portion R and indirectly onto the central portion C. The wings 24 are folded onto the front and rear portions F, R. As illustrated in FIG. 3, the wings 24 are folded directly onto the rear portion R. As illustrated in FIG. 3, the wings 24 are folded indirectly onto the front and central portions F,C. Hence, with respect to the vertical direction V, the sanitary napkin 10 in a final or fully folded configuration, is arranged with the front and rear portions F,R being arranged between the central portion C and the wings 24. As illustrated in FIG. 3, the sanitary napkin 10 is folded in three, meaning comprising two folding lines 29, and is maintained in a folded configuration, or folded state, or folded conformation, by the wings 24. In order words, the wings 24 acts as clamping mean maintaining the sanitary napkin 10 in a folded state. The wings 24 are overlaying the front and rear portions F,R and the central portion C is underlaying the front and rear portions F,R.

In other to maintain the sanitary napkin 10 in a folded state, the sanitary napkin 10 can comprise additional means to ensure this clamping means.

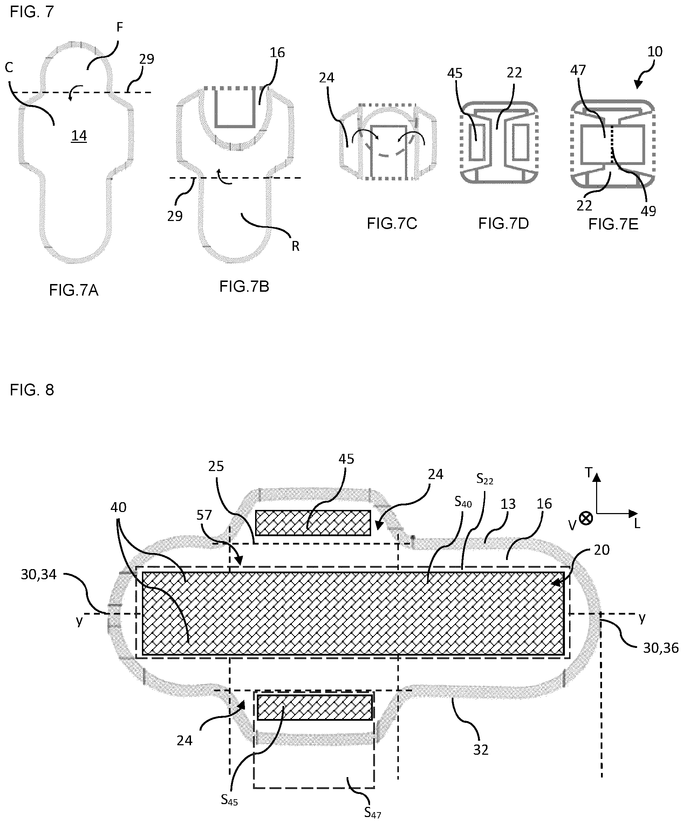

For example, as illustrated in FIG. 4, the sanitary napkin 10 comprises one removable release liner 47 connecting both wings 24, meaning a removable release liner 47 covering the second area of adhesive 45 meaning covering both patch or strips of adhesive material 45 arranged on both wings. The removable release liner 47 can optionally comprise pre-cuts 49 or perforations 49 or weakened division line 49 as illustrated in FIG. 7E in order to ease the removal of the release liner 47.

According to another example as illustrated in FIG. 5, the wings 24 overlap at least partially, once said wings 24 are folded onto the absorbent core and rear portion R. For example the wings 24 can be of different size, with one wing 24 being longer than the other wing 24, or the wings 24 can both extend in the transversal direction T on a width that is greater than the width of the main body 57, meaning the total width of the two wings 24 is greater than the total width of the main body 57. Such arrangement comprises one overlapping or overlaying wing 24 and another underlaying wing 24. The overlapping wing 24 comprises a removable release liner 47 covering the second area of adhesive 45 arranged on the overlapping wing 24 and the other wing 24, *i.e.* the underlaying wing 24, either comprising a removable release liner 47 covering the second area of adhesive 45 arranged on the underlying wing 24 or the overlapping wing 24 covers the second area of adhesive 45 arranged on the underlying wing 24

According to another example as illustrated in FIG. 6, the wings 24 can be attached and comprise a breakable bond, for example pre-cuts 49 or perforations 49, i.e. a weakened division line 49. Each wing 24 comprising a removable release liner 47 covering the second area of adhesive 45, meaning covering the patch or strips of adhesive material 45 arranged on each wing 24.

The present disclosure also pertains to a method for folding a sanitary napkin 10 as described herein, the method comprises the following steps:
- folding the front portion (F) onto the central portion (C) and folding the rear portion (R) onto the front portion (F) ; or
- folding the rear portion (R) onto the central portion (C) and folding the front portion (F) onto the rear portion; and
- Folding the wings 24 onto the folded front and rear portions (F,R).

FIG. 7 (7A to 7E) exemplifies one example of the method according to a top-view, or when viewing the sanitary napkin 10 from above, the method comprises the following steps:
- folding the front portion (F) onto the central portion (C) (FIG. 7A to 7B), the front portion F being folded around a folding line 29; namely the folding line 29 dividing the front and central portions F,C;
- folding the rear portion (R) onto the front portion F (Fig. 7B to 7C), the rear portion R being folded around another folding line 29; namely the folding line 29 dividing the rear and central portions R,C;
- folding both wing 24, either sequentially or simultaneously, onto the folded front and rear portions (F,R), here directly over the rear portion (R), more specifically, the wings 24 are folded onto the portion, front or rear, which was folded last. (FIG. 7C to 7D).

The front and/or rear portions F,R are folded inwardly, meaning towards the tospheet 14 or towards the side of the sanitary napkin 10 where the tophseet is arranged. In other words, the front and/or rear portions F,R are folded towards the body-facing side 17.

The present disclosure is not limited to this illustrated example, for example, the first step can comprise the folding the rear portion (R) onto the central portion (C) and then the folding of the front portion onto the rear portion.

According to the embodiment of FIG. 7, applying a removable release liner 47 onto the second area of adhesive 45 arranged on each wing 24, the first area of adhesive being covered by the release paper 22.

When seeing the absorbent article 10, or sanitary napkin 10, as illustrated in FIG. 7E, it is apparent that such sanitary napkin 10 corresponds to a more environmentally friendly absorbent article. Such sanitary napkin 10 is free of wrapping plastic film or plastic pouch and the topsheet 14 and absorbent core 12, meaning the portion of the absorbent article that is in contact with the wearer's body is well protected by the backsheet 16 and the release paper 22. The topsheet 14 is also well protected since the wings 24 and eventual release liner 47 maintain the absorbent article 10 in a folded configuration.

Hence wings 24 according to the present disclosure still acts as a clamping mean, or a holding mean, for the sanitary napkin 10 and keeps it in a folded configuration while saving on material and thus being more environmentally friendly. Additionally, by not using a conventional pouch, there is no need to add a closing tape with the present assembly 1 again saving on material.

According to a further embodiment, the release paper 22, release liner 47 and/or the backsheet 16 can also comprise a graphic element printed upon its surface. The graphic elements on the release paper 22, release liner 47 and/or backsheet 16, can be matching or congruent. By congruent it is meant capable of being placed over another figure and exactly matching, for example if a circle is printed on the release paper 22, the backsheet 16 or release liner 47 comprises two arcuate lines matching the partial periphery of said circle. The graphic element printed on the release paper 22 and/or backsheet 16 can be of the same nature as the graphic element printed on the release liner 47 or of different nature. For example the release paper 22 and/or backsheet 16 can comprise a pattern printed upon its outer surface whereas the wrapper comprises one or more words printed upon its outer surface or the example the release paper 22 and/or backsheet 16 and the wrapper can all comprise a drawing printed upon its surface. According to a further embodiment, the release paper 22, the backsheet 16 and the release liner 47 each comprises a graphic element.

The topsheet 14 is protected by the release paper 22 and the wings 24 as show in FIG. 4. In a folded configuration, the total surface area of the wings 24 and the folded release paper 22 covers preferably between 50% and 100 % of the total surface area of the folded absorbent article 10, more preferably, between 60% and 99%, even more preferably, between 70% and 98%. For sake of clarity, in a final folded configuration, the total apparent surface area of the release paper 22 and wings 24 covers preferably between 50% and 100 % of the total apparent surface area of the absorbent article 10, more preferably, between 60% and 99%, even more preferably, between 70% and 98%. The topsheet 14 is already well protected and using wings and release paper 22 covering between 60 and 99% of the total surface area of the folded absorbent article 10 enables to save on material.

As described above, the release paper 22 and/or release liner 47 are preferably siliconized paper with a paper layer is coated with a layer of peeling agent selected from silicon resin series, fluororesin series, octadecilisocyanate series. According to an embodiment, the peeling layer comprises a basis weight comprised between 0,3 gsm and 3 gsm, preferably between 0,5 gsm and 1 gsm and the paper layer comprises a basis weight comprised between 10 gsm and 80 gsm, preferably between 12 and 60 gsm. This release paper 22 and/or release line 47 are preferably recyclable.

The layer of adhesive 20 comprises the first area of adhesive 40 and second area of adhesive 45 preferably comprises pressure sensitive adhesive as such adhesive is easy to attach and remove from the garment. Example of pressure sensitive adhesive comprises and are not limited to styrene block copolymers (SBC), solvent-acrylic, emulsion-acrylic, silicone or solvent-rubber based material. The player of adhesive 20 can comprise other types of adhesive such as non-petroleum derived material as to lower the environmental impact of the absorbent article. The adhesive are preferably selected from and not limited to polylactic (PLA), 3-hydroxy butyric acid and 3-hydroxy pentanoic acid (3-hydroxy valeric acid) (PHBV), polyvinyl alcohol (PVOH), biobased ethylene vinyl acetate (EVA), polyurethane (PU), vinyl acetate polymers (PVA) polyethylene oxide (PEO), polyhydroxylakanoate (PHA), polycaprolactone (PCL), a polymer material such as a polyethylene material, e.g. as a low-density polyethylene (LDPE), a bio coating, a printed lacquer, 1,4 succinic acid, fumaric acid, malic acid, 2,5 furan dicarboxylic acid, 3 hydroxy propionic acid, aspartic acid, glucaric acid, glutamic acid, itaconic acid, levulinic acid, 3-hydroxybutyrolactone, glycerol, sorbitol, xylitol/arabinitol or tricarboxylic acid. The adhesive and/or heat-sealable material preferably comes from a sustainable source, or bio-based or bioplastic, and is biodegradable and/or compostable.

Given that the objective is to reduce the environmental impact of the assembly 1 comprising an absorbent article 10 and a release paper 22, the release paper 22 can be partially coated, such that the coating of peeling agent is equal to or slightly greater than the first area of adhesive 40. According to another embodiment, the release paper 22 and/or the release liner 47 is partially coated such that the coating of peeling agent is equal or slightly greater than the first area of adhesive 40 and/or second area of adhesive 45 respectively. As shown in FIG. 2, when the layer of adhesive 20, namely the first and/or second area of adhesive 40,45, is applied in a discontinuous pattern, the release paper 22 will therefore cover zones of the backsheet 16 that does not comprise any adhesive. It can be beneficial to have a release paper 22 that is not fully coated with peeling agent thereby saving on raw material, namely peeling agent such as silicone.

Alternatively or in addition, again given that the objective is to reduce the environmental impact of the assembly 1 comprising an absorbent article 10 and a release paper 22, the release paper 22 of reduced dimensions such that the surface area of the release paper is slightly greater than the first area of adhesive 40. According to another embodiment, the release paper 22 and/or the release liner 47 is/are of dimensions such that the surface area of the release paper 22 and/or the release liner 47 are equal or slightly greater than the first area of adhesive 40 and/or second area of adhesive 45 respectively. Indeed, it can be beneficial to have a release paper 22 and/or release liner 47 of reduced dimensions.

As seen above, the release paper 22 itself can reduce the environmental impact of the assembly by using less material, i.e. less silicone. According to another aspect of the present disclosure, the present disclosure also pertains to an assembly 1 comprising a release paper 22 and an absorbent article 10, the absorbent article 10 comprising a topsheet 14, a backsheet 16, an absorbent core 12 arranged between said topsheet 14 and backsheet 16, and a layer of adhesive 20 being arranged on a side of the backsheet 16 opposite to the absorbent core 12, the release paper 22 being arranged on a side of the layer of adhesive 20 opposite to the backsheet 16 wherein the release paper 22 is partially coated by a coating of peeling agent, such that the coating of peeling agent is equal to or slightly greater than the first area of adhesive 40. The following is with respect to this other aspect of the present disclosure and can be applied to an absorbent article 10 with wings such as a sanitary napkin or a wingless absorbent article 10 such as a incontinence pad or a pantyliner.

According to an embodiment, the release paper 22 and/or the release liner 47 is partially coated such that the coating of peeling agent is equal or slightly greater than the first area of adhesive 40 and/or second area of adhesive 45 respectively. When the layer of adhesive 20, namely the first and/or second area of adhesive 40,45, is applied in a discontinuous pattern, the release paper 22 will therefore cover zones of the backsheet 16 that does not comprise any adhesive. It can be beneficial to have a release paper 22 that is not fully coated with peeling agent thereby saving on raw material, namely peeling agent such as silicone. the release paper 22 and/or release liner 47 of the present disclosure has peelable properties with an area of a surface of the release paper 22 and/or release liner 47 being subjected to peeling treatment(s) as described hereabove. For example, the area of the surface of the release paper 22 and/or release liner 47 with which the layer of adhesive 20 of the absorbent article 10 is in contact, and eventually its vicinity, can be subjected to said peeling treatment(s) namely the coating of a layer of peeling agent such as silicone. For example, the layer of adhesive 20 is applied continuously between the front, central and/or rear portions F,C,R in two or more stripes extending parallel and elongated in the longitudinal direction, the release paper 22 can be as short and/or as thin than these stripes of adhesive material or the release paper 22 can be wider and/or longer than these stripes of adhesive material but comprise a peeling coating that is as short and/or as thin than these stripes of adhesive material, or in other words, the dimensions, namely the length and width, of the release paper and/or coating of peeling agent is equal to the dimensions, namely the length and width, of the stripes of adhesive material. Similarly, the dimensions, namely the length and width, of the release liner and/or coating of peeling agent is equal to the dimensions, namely the length and width, of the stripes of adhesive material on the wings. In other words, the release paper and the adhesive on the backsheet can be congruent, meaning are of same size and shape and are placed one over the other and exactly matching.

The area of peeling agent can be equal to or slightly greater than the area defined by the layer of adhesive 20. As seen above, the layer of adhesive 20 is applied discontinuously on a portion of the backsheet 16 of the absorbent article 10. The area of peeling agent can also be arranged, or applied or coated, discontinuously on a surface of the release paper 22 and/or release liner 47. In other words, the adhesive can be applied on the backsheet 16 in a discontinuous pattern, same as the peeling agent which can be applied in a discontinuous pattern on the release paper 22. For example, the area of peeling agent 32 is greater than 30%, preferably comprised between 40% and 95%, more preferably between 50% and 90% of the total area of a surface of the release paper 22 and/or release liner 47.

According to an embodiment, the ratio between the total surface area of peeling agent arranged on the release paper 22 and/or release liner 47 and the total surface area of adhesive arranged on the backsheet 16 is comprised between 1 and 1.5, preferably between 1 and 1.2, more preferably between 1 and 1.1, even more preferably between 1 and 1.05, even more preferably said ratio is equal to 1. For example, the ratio between the total surface area of peeling agent arranged on the release paper 22 and the total surface area of adhesive arranged on the main body 57 region is 1.4 and/or the ratio between the total surface area of peeling agent arranged on each release liner 47 and the total surface area of adhesive 45 arranged on each wings 47 is 1.2. According to these embodiments, the area, or surface area, of peeling agent is preferably arranged discontinuously, meaning the peeling agent is arranged in a discontinuous pattern, on a surface of the release paper 22 and/or release liner 47, similarly, the area of adhesive is preferably arranged discontinuously, meaning the adhesive is arranged in a discontinuous pattern, on a surface of the backsheet 16. In other words, the peeling agent coated on the release paper 22 and the adhesive on the backsheet 16, meaning the first and/or second area of adhesive 40,45, are congruent, meaning are of same size and shape and are placed one over the other and exactly matching.

According to an embodiment, the ratio between the total surface area of the release paper 22 and the total surface area of the first area of adhesive 40 and arranged on the backsheet 16, namely in the main body 57 region, is comprised between 1 and 2.5, preferably between 1 and 2.0, more preferably between 1 and 1.8, even more preferably between 1.05 and 1.6, even more preferably said ratio is comprised between 1.1 and 1.5. According to these embodiments, the area of adhesive arranged on the backsheet 16 can be arranged continuously, meaning forming a continuity of matter, or discontinuously. In other words, the adhesive can be applied on the backsheet 16 in a continuous pattern. For example, the layer of adhesive 20 is applied continuously between the front, central and/or rear portions F,C,R in two or more stripes extending parallel and elongated in the longitudinal direction, the release paper 22 can be as short and/or as thin than these stripes of adhesive material or the release paper 22 can be wider and/or longer than these stripes of adhesive material but comprise a peeling coating that is as short and/or as thin than these stripes of adhesive material, or in other words, the dimensions, namely the length and width, of the release paper and/or coating of peeling agent is equal to the dimensions, namely the length and width, of the stripes of adhesive material. Similarly, the dimensions, namely the length and width, of the release liner and/or coating of peeling agent is equal to the dimensions, namely the length and width, of the stripes of adhesive material on the wings.

For example, taking FIG. 2, the surface area S₄₀ of the first area of adhesive 40 is defined by the addition of the continuous surface areas of adhesive material, here two stripes, on the main body 57 region. The surface area S₂₂ of the release paper is also illustrated in dashes in FIG. 2. When the adhesive is applied in multiple stripes as in FIG. 2, the ratio between the total surface area S₂₂ of the release paper 22 and the total surface area S₄₀ of the first area of adhesive 40 is comprised between 1.05 and 2.5, preferably between 1.2 and 2.0, more preferably between 1.45 and 1.9. When the adhesive is applied in one bloc as in FIG. 8, the ratio between the total surface area S₂₂ of the release paper 22 and the total surface area of the first area of adhesive 40 is comprised between 1.05 and 2.0, preferably between 1.1 and 1.8, more preferably between 1.3 and 1.7. Simply put the ratio S₂₂/S₄₀ is comprised between 1.05 and 2.5 as described hereabove. For example when the first area of adhesive 40 corresponds to two stripes of adhesive material, the release paper 22 can comprise 2 stripes of release paper 22 of similar dimensions as the stripes of adhesive material and have a ratio S₂₂/S₄₀ comprised between 1.05 and 1.1. Preferably the surface area of the release paper 22 is greater than the surface area of the first area of adhesive 40 so that the release paper 22 can be easily grasped and peeled off.

As described above, the present disclosure is not limited to these embodiments, for example it is possible to combine the ratio between the surface area of the release paper and the surface area of peeling agent on said release paper with the ratio between the surface area of the release paper and the surface area of the first area of adhesive 40.

For example, the absorbent article 10, or the sanitary napkin, comprises a release paper 22 such that the ratio between the total surface area of peeling agent arranged on the release paper 22 and/or release liner 47 and the total surface area of adhesive arranged on the backsheet 16 is comprised between 1 and 1.5 and the ratio between the total surface area of the release paper 22 and the total surface area of the first area of adhesive 40 arranged on the backsheet 16, namely in the main body 57 region, is comprised between 1.0 and 2.5, preferably between 1.0 and 2.0, more preferably between 1.0 and 1.8, even more preferably between 1.05 and 1.6, even more preferably said ratio is comprised between 1.1 and 1.5.

The present disclosure pertains to an absorbent article 10 comprising a topsheet 14, a backsheet 16, an absorbent core 12 arranged between said topsheet 14 and backsheet 16, a layer of adhesive 20 being arranged on a side of the backsheet 16 opposite to the absorbent core 12 and a release paper 22 being arranged on a side of the layer of adhesive 20 opposite to the backsheet 16, wherein the ratio between the total surface area of the release paper 22 and the total surface area of the first area of adhesive 40 arranged on the backsheet 16, namely in the main body 57 region, is comprised between 1.0 and 2.5, preferably between 1.0 and 2.0, more preferably between 1.0 and 1.8, even more preferably between 1.05 and 1.6, even more preferably said ratio is comprised between 1.1 and 1.5.

The present disclosure pertains to an absorbent article 10 comprising a topsheet 14, a backsheet 16, an absorbent core 12 arranged between said topsheet 14 and backsheet 16, a layer of adhesive 20 being arranged on a side of the backsheet 16 opposite to the absorbent core 12 and a release paper 22 being arranged on a side of the layer of adhesive 20 opposite to the backsheet 16, wherein the ratio between the total surface area of peeling agent arranged on the release paper 22 and the total surface area of adhesive arranged on the backsheet 16 is comprised between 1.0 and 1.5 and the ratio between the total surface area of the release paper 22 and the total surface area of the first area of adhesive 40 arranged on the backsheet 16, namely in the main body 57 region, is comprised between 1.0 and 2.5, preferably between 1.0 and 2.0, more preferably between 1.0 and 1.8, even more preferably between 1.05 and 1.6, even more preferably said ratio is comprised between 1.1 and 1.5.

Similarly, the ratio between the total surface area of the release liner 47 and the total surface area of the second area of adhesive 45 arranged on the backsheet 16, namely in the main wings 24, is comprised between 1 and 2.5, preferably between 1.1 and 2.4, more preferably between 1.2 and 2.2, even more preferably between 1.3 and 2.1, even more preferably said ratio is comprised between 1.5 and 2.0. According to these embodiments, the area of adhesive arranged on the wings 24 can be arranged continuously, meaning forming a continuity of matter, or discontinuously.

For example, taking FIG. 8, the surface area S₄₅ of the second area of adhesive 45 is defined by the addition of the continuous surface areas of adhesive material, here two stripes, on both wings 24. The surface area S₄₇ of the release liner is also illustrated in dashes in FIG. 8. Simply put the ratio S₄₇/S₄₅ is comprised between 1.0 and 2.5 as described hereabove.

The present disclosure pertains to an absorbent article 10 comprising a topsheet 14, a backsheet 16, an absorbent core 12 arranged between said topsheet 14 and backsheet 16, wings 24, a layer of adhesive 20 being arranged on a side of the backsheet 16 opposite to the absorbent core 12 and a release paper 22 being arranged on a side of the layer of adhesive 20 opposite to the backsheet 16 and release liner(s) 47 being arranged on a side of the layer of adhesive 20 opposite to the backsheet 16, wherein the ratio between the total surface area of the release paper 22 and the total surface area of the first area of adhesive 40 arranged on the backsheet 16, namely in the main body 57 region, is comprised between 1.0 and 2.5, preferably between 1.0 and 2.0, more preferably between 1.0 and 1.8, even more preferably between 1.05 and 1.6, even more preferably said ratio is comprised between 1.1 and 1.5. Alternatively or in addition the ratio between the total surface area of the release liner 47 and the total surface area of the second area of adhesive 45 arranged on the backsheet 16, namely in the main wings 24, is comprised between 1 and 2.5, preferably between 1.1 and 2.4, more preferably between 1.2 and 2.2, even more preferably between 1.3 and 2.1, even more preferably said ratio is comprised between 1.5 and 2.0.

The present disclosure pertains to an absorbent article 10 comprising a topsheet 14, a backsheet 16, an absorbent core 12 arranged between said topsheet 14 and backsheet 16, a layer of adhesive 20 being arranged on a side of the backsheet 16 opposite to the absorbent core 12 and a release paper 22 being arranged on a side of the layer of adhesive 20 opposite to the backsheet 16, wherein the ratio between the total surface area of peeling agent arranged on the release paper 22 and the total surface area of adhesive arranged on the backsheet 16 is comprised between 1.0 and 1.5 and the ratio between the total surface area of the release paper 22 and the total surface area of the first area of adhesive 40 arranged on the backsheet 16, namely in the main body 57 region, is comprised between 1.0 and 2.5, preferably between 1.0 and 2.0, more preferably between 1.0 and 1.8, even more preferably between 1.05 and 1.6, even more preferably said ratio is comprised between 1.1 and 1.5. Alternatively or in addition the ratio between the total surface area of the release liner 47 and the total surface area of the second area of adhesive 45 arranged on the backsheet 16, namely in the main wings 24, is comprised between 1 and 2.5, preferably between 1.1 and 2.4, more preferably between 1.2 and 2.2, even more preferably between 1.3 and 2.1, even more preferably said ratio is comprised between 1.5 and 2.0.

It is possible to combine the features of the different embodiments mentioned hereabove. For example a release line 47 can comprise a graphic element congruent or matching the graphic element of the release paper 22 and pre-cuts 49 i.e. a weakened division line 49. To have a sanitary napkin 10 as described hereabove is arranged in a folded configuration such that the absorbent core is maintained in said folded configuration by the wings 24 as described above and a layer of adhesive 20 being arranged on a side of the backsheet 16 opposite to the absorbent core 12 and a release paper 22 being arranged on a side of the layer of adhesive 20 opposite to the backsheet 16 and release liner(s) 47 being arranged on a side of the layer of adhesive 20 opposite to the backsheet 16, wherein the ratio between the total surface area of the release paper 22 and the total surface area of the first area of adhesive 40 arranged on the backsheet 16, namely in the main body 57 region, is comprised between 1.0 and 2.5, preferably between 1.0 and 2.0, more preferably between 1.0 and 1.8, even more preferably between 1.05 and 1.6, even more preferably said ratio is comprised between 1.1 and 1.5 or alternatively or in addition the ratio between the total surface area of the release liner 47 and the total surface area of the second area of adhesive 45 arranged on the backsheet 16, namely in the main wings 24, is comprised between 1 and 2.5, preferably between 1.1 and 2.4, more preferably between 1.2 and 2.2, even more preferably between 1.3 and 2.1, even more preferably said ratio is comprised between 1.5 and 2.0. It is supposed that the present invention is not restricted to any form of realization described previously and that some modifications can be added to the presented example of fabrication without reappraisal of the appended claims.

## Claims

1. Sanitary napkin (10) comprising :
- a topsheet (14),
- a backsheet (16),
- an absorbent core (12) arranged between said topsheet (14) and backsheet (16), and
- two wings (24),
wherein the sanitary napkin (10) is arranged in a folded configuration such that the absorbent core is maintained in said folded configuration by the wings (24).

2. Sanitary napkin (10) according to claim 1, wherein the sanitary napkin (10) extends in longitudinal, transversal and vertical directions (L,T,V), and comprises in a unfolded configuration with respect to the longitudinal direction, a front portion (F), a rear portion (R) and a central portion (C) arranged between said front and rear portions (F,R), the central portion (C) being defined preferably substantially by the length of the wings (24) taken in the point in which they extend from the longitudinal sides (32) of the sanitary napkin (10) in a direction parallel to the longitudinal axis (y-y), when in a said folded configuration, the front and rear portions (F,R) are folded onto the central portion (C), the front and rear portions (F,R) being arranged between the central portion (C) and the wings with respect to the vertical direction (V)

3. Sanitary napkin (10) according to any of the preceding claims, wherein each wing (24) comprises an area of adhesive (45) arranged on the side of the backsheet (16) opposite to the absorbent core (12).

4. Sanitary napkin (10) according to claim 3, wherein the sanitary napkin (10) comprises a release liner (47) covering both areas of adhesives (45) arranged on the wings (24).

5. Sanitary napkin (10) according to any of the claims 1 to 3, wherein the wings (24) are at least partially overlapping when folded onto the front or rear portion (F,R), preferably said overlapping portion comprising adhesive to attach one wing (24) to the other.

6. Sanitary napkin (10) according to any of the preceding claims, wherein a layer of adhesive (20) is arranged on the side of the backsheet (16) opposite to the absorbent core (12), a release paper (22) being applied onto said layer of adhesive in a main body (57) region arranged inboard of the wings (24) with respect to the transversal direction.

7. Sanitary napkin (10) according to any of the claims 4 or 6, wherein the release paper (22) and/or release liner (47) comprises a paper layer comprising cellulosic fibers and a coating comprising a peeling agent selected from silicon resin series, fluororesin series, octadecilisocyanate series wherein said paper layer comprises a basis weight comprised between 10 gsm and 80 gsm, preferably between 12 and 60 gsm.

8. Sanitary napkin (10) according to claim 6 or to claims 6 and 7 wherein in a folded configuration the release paper (22) and wings (24) cover between 50 and 100 % of the backsheet located in a main body region of the sanitary napkin (10) arranged inboard of the wings (24) with respect to the transversal direction.

9. Sanitary napkin (10) according to claim 6 or any claim dependent of claim 6, wherein the ratio between the total surface area (S₂₂) of the release paper (22) and the total surface area (S₄₀) of the first area of adhesive (40) is comprised between 1.05 and 2.5.

10. Sanitary napkin (10) according to claims 6 and 7 or any claim dependent of claims 6 and 7, wherein the ratio between the total surface area of peeling agent arranged on the release paper (22) and/or release liner (47) and the total surface area of adhesive arranged on the backsheet (16) is comprised between 1 and 1.5.

11. Method for folding a sanitary napkin (10) according to any of the preceding claims, wherein said method comprises the following steps :
- folding the front portion (F) onto the central portion (C) and folding the rear portion (R) onto the front portion (F) ; or
- folding the rear portion (R) onto the central portion (C) and folding the front portion (F) onto the rear portion; and
- folding the wings (24) onto the folded front and rear portions (F,R).

12. Method according to claim 11 dependent of claim 4 and following, wherein the method comprises a further step of applying a release liner (47) onto both areas of adhesive arranged on the wings (24).

13. A container comprising a plurality of folded sanitary napkin (10) according to any of the claims 1 to 10, said container being selected from a reusable bag, a paperbag, a cardboard box or a metal box
